(19) Europäisches Patentamt
European Patent Office
Office européen des brevets

(11) Veröffentlichungsnummer: **0 033 880**
**B1**

(12) # EUROPÄISCHE PATENTSCHRIFT

(45) Veröffentlichungstag der Patentschrift: **02.03.83**

(51) Int. Cl.³: **C 07 C 47/04,** C 07 C 45/00, B 01 J 23/80, B 01 J 27/02

(21) Anmeldenummer: **81100527.1**

(22) Anmeldetag: **24.01.81**

(54) **Verfahren zur Herstellung von Formaldehyd.**

(30) Priorität: **07.02.80 DE 3004436**

(43) Veröffentlichungstag der Anmeldung: **19.08.81 Patentblatt 81/33**

(45) Bekanntmachung des Hinweises auf die Patenterteilung: **02.03.83 Patentblatt 83/9**

(84) Benannte Vertragsstaaten: **BE CH DE FR GB LI NL**

(56) Entgegenhaltungen:
**DE-A-2 525 174**
**DE-A-2 627 421**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Fouquet, Gerd, Dr., Maxburgstrasse 2, D-6730 Neustadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 033 880 B1

BUNDESDRUCKEREI BERLIN

## Verfahren zur Herstellung von Formaldehyd

Die Erfindung betrifft ein Verfahren zur Herstellung von Formaldehyd durch Dehydrierung von Methanol in Gegenwart von Kupfer, Zink und Tellur enthaltenden Katalysatoren bei einer Temperatur von 300 bis 750°C.

Es ist aus der deutschen Auslegeschrift 1 144 252 und aus Ullmanns Encyklopädie der technischen Chemie (4. Auflage), Band 11, Seiten 693 bis 696, bekannt, Methanol in Gegenwart von Metalloxidkatalysatoren, in der Regel an Eisenoxid und Molybdänoxid ohne Träger zu Formaldehyd zu oxidieren. Einen entsprechenden Katalysator, der noch dazu Kobaltoxid enthält, schildert die deutsche Patentschrift 1 162 344. Die deutsche Auslegeschrift 1 063 141 lehrt die Oxidation in Gegenwart solcher Katalysatoren auf einem Träger, wobei Carbid- und/oder Eisenoxid-Träger verwendet werden. Es werden Temperaturen von 270 bis 380°C angegeben. Alle diese Verfahren liefern mittels katalytischer Oxidation 37- bis 55-gewichtsprozentige, wäßrige Formaldehydlösungen. Die Lagerung und Transport solcher Lösungen bereiten Schwierigkeiten, da Paraformaldehydniederschläge und entsprechende Ablagerungen und Verstopfungen in den Anlagen auftreten, sofern nicht Stabilisatoren bzw. erhöhte Lagertemperaturen angewendet werden. Erhöhte Lagertemperaturen begünstigen die Bildung von Ameisensäure. Wasserarmer methanolischer Formaldehyd wird nach diesen Verfahren nicht hergestellt.

Aus diesen Gründen wurden schon Verfahren zur Herstellung von wasserarmen Formaldehydlösungen mit Kupfer/Silber/Silicium bzw. Kupfer/Zink bzw. Zink/Gallium/Indium als Katalysatoren, Aluminiumkatalysatoren und Zinkkatalysatoren entwickelt, die in der deutschen Offenlegungsschrift 2 525 174 beschrieben werden. Als vorteilhafter wird eine Dehydrierung von Methanoldampf im Gemisch mit Wasserstoff und Kupfer, Zink und Schwefel als Katalysatorkomponenten bei 500 bis 750°C angesehen. In der deutschen Offenlegungsschrift 2 627 421 wird eine ähnliche Verfahrensweise, die anstelle von Schwefel Selen als Katalysatorkomponente verwendet, beschrieben. Es wird angegeben, daß Anteile an Schwefel im Katalysator bei der Dehydrierung unerwünschterweise von dem Reaktionsprodukt oder von austretendem Gas mitgerissen werden. Nachteilig bei dem Verfahren der deutschen Offenlegungsschrift 2 627 421 ist, daß auch Selen während der Reaktion mitgerissen und der Katalysator bei längerem Betrieb zerstört wird. Insbesondere ist nachteilig, daß Schwefel und Selen, insbesondere in Form ihrer Wasserstoffsäuren, den Endstoff verunreinigen und im Abgas Umweltschutzprobleme verursachen. Der verunreinigte Endstoff liefert entsprechende Schwierigkeiten bei seiner Weiterverarbeitung. Insgesamt werden zusätzliche Maßnahmen und Anlagen mit Bezug auf Aufarbeitung, Reinigung des Endstoffs und des Abgases sowie Regelung und Kontrolle der Anlagen notwendig.

Es wurde nun gefunden, daß man Formaldehyd durch Dehydrierung von Methanol in Gegenwart eines Katalysators bei erhöhter Temperatur vorteilhaft erhält, wenn die Umsetzung in Gegenwart von Kupfer, Zink und Tellur enthaltenden Katalysatoren bei einer Temperatur von 300 bis 750°C durchgeführt wird.

Die Umsetzung kann durch die folgenden Formeln wiedergegeben werden:

$$CH_3OH \rightarrow CH_2O + H_2$$

Im Vergleich zu den bekannten Verfahren liefert das Verfahren nach der Erfindung überraschend auf einfacherem und wirtschaftlicherem Wege Formaldehyd in guter Ausbeute und Reinheit. Im Hinblick auf die bekannten Verfahren, die zu wäßrigen Formaldehydlösungen führen, erhält man wasserarme, methanolische, zweckmäßig 10- bis 40-gewichtsprozentige Formaldehydlösungen. Der Wassergehalt liegt in der Regel unter 1,5 Gewichtsprozent, meist von 0,2 bis 0,9 Gewichtsprozent, bezogen auf die Gesamtlösung. Der Katalysator hat eine wesentlich höhere Lebensdauer und Aktivität, meist über oder gleich 80 Betriebsstunden im Vergleich zu weniger oder gleich 10 Betriebsstunden im Falle Kupfer/Zink/Schwefel und weniger oder gleich 40 Betriebsstunden im Falle Kupfer/Zink/Selen als Katalysator bei vergleichbaren Umsätzen. Der Zusatz von Wasserstoffgas kann überraschend eingespart werden. Es war im Hinblick auf vorgenannte Veröffentlichungen nicht zu erwarten, daß Tellur bei der Reaktion kaum vom Katalysator entfernt wird. Der Gehalt des Reaktionsgemisches vergleichbarer Umsetzungen beträgt in der Regel $\leqq$ 0,5 mg/kg Tellur, 3 bis 175 mg/kg S bei schwefelhaltigen und 3 bis 830 mg/kg Se bei selenhaltigen Katalysatoren. Entsprechend ist das Verfahren betriebssicherer und umweltfreundlicher und erspart zusätzliche Kontroll- und Reinigungsoperationen, Anlagen und häufigen Katalysatorwechsel. Alle diese vorteilhaften Ergebnisse des erfindungsgemäßen Verfahrens sind insbesondere auch im Hinblick auf J. Falbe und U. Hasserodt, Katalysatoren, Tenside und Mineralöladditive (Thieme Verlag, Stuttgart 1978), Seite 67, überraschend, da dort ausdrücklich erklärt wird, daß Kontakte zur Oxidation von Acrolein zu Acrylsäure unter Verwendung von Kobalt-, Molybdän- und Telluroxid gerade wegen der Flüchtigkeit des Tellurs keine technische Anwendung gefunden haben.

Für das Verfahren geeignete Ausgangsstoffe sind reines Methanol oder technisches Methanol. Auch Rohmethanol, das in der Regel nach den in DAS 1 277 834 und DP 1 235 881 beschriebenen Verfahren durch Behandlung mit Oxidationsmitteln und/oder Alkalien gereinigt wird, kann verwendet werden.

Der Katalysator enthält die Metalle zweckmäßig in einem Atomverhältnis von 1 Kupfer zu 0,01 bis 0,5, vorzugsweise 0,05 bis 0,4 Zink und zu 0,001 bis 0,3, vorzugsweise 0,005 bis 0,2 Tellur. Vorteilhaft ist ein Atomverhältnis 0,05 bis 0,3 Zink zu 1 Kupfer und/oder 0,005 bis 0,1 Tellur zu 1 Kupfer. Die vorgenannten Elemente im Katalysator werden hier ohne Berücksichtigung ihrer tatsächlichen Konstitution bezeichnet und berechnet. Die Elemente können im Katalysator während der Reaktion als Metalle und/oder Metallverbindungen vorliegen, zweckmäßig wird der Ausgangskatalysator nur die Metallverbindungen enthalten, die im Laufe der Reaktion teilweise zum Metall reduziert werden. Vorteilhaft wählt man Katalysatoren, deren Metallverbindungen am Ende der Reaktion 94,2 bis 66,2 Gewichtsprozent des Gesamtkupfers in Gestalt von metallischem Kupfer, 4,9 bis 20,5 Gewichtsprozent des Gesamtzinks in Gestalt des metallischen Zinks und 0,9 bis 13,3 Gewichtsprozent des Gesamttellurs in Gestalt von elementarem Tellur enthalten.

Als Verbindungen der Metalle kommen in Frage: die Hydroxide, Oxide, Nitrate, Bicarbonate, Carbonate, Acetate, Oxalate des Kupfers, Zinks oder Tellurs; Sauerstoffsäure des Tellurs, Tellurate wie Ammonium-oder Kaliumtellurat und entsprechende Tellurite; Kupfertellurat, Zinktellurat, Kupfertellurit, Zinktellurit.

Zweckmäßig ist ein Verhältnis im Katalysator von 10 bis 50, vorzugsweise 12 bis 40 Grammatom Kupfer, 0,5 bis 20, vorzugsweise 1 bis 15 Grammatom Zink und 0,1 bis 10, vorzugsweise 0,3 bis 8 Grammatom Tellur je Mol Methanol bzw. je Mol $CH_3OH$ und Stunde. Der Katalysator besteht in der Regel aus Teilchen beliebiger, aber vorzugsweise kugeliger Form, mit einem Durchmesser von 2 bis 12, vorzugsweise von 3 bis 10 Millimeter. Es kann in üblicher Weise, z. B. durch Vermischen der Kupfer-, Zink- und Tellurverbindungen mit wenig Wasser und Trocknen bei 100 bis 140°C, hergestellt werden. Man kann auch den Katalysator durch Erhitzen, z. B. während 2 bis 4 Stunden zuerst bei einer Temperatur zwischen 100 und 140°C und dann zwischen 140 und 750°C trocknen und aktivieren, wobei die Verbindungen ganz oder teilweise in die Oxide umgewandelt werden.

Die Dehydrierung des Methanols wird bei einer Temperatur von 300 bis 750°C, vorzugsweise zwischen 320 und 600°C, drucklos oder unter Druck, diskontinuierlich oder vorzugsweise kontinuierlich durchgeführt. Die Reaktion kann wie folgt gestaltet werden: Ein Reaktor wird mit dem Katalysator gefüllt. Vorteilhaft verwendet man als Reaktor einen Röhrenreaktor mit äußerer Kühlung und einer beliebigen Anzahl von Rohren, z. B. mit 8000 bis 15 000 Röhren, die zweckmäßig frei von Einbauten, z. B. Wärmeschächten, s' In einer bevorzugten Ausführungsform beträgt der innere Durchmesser der Rohre mindestens 25 Millimeter, insbesondere 25 bis 34 Millimeter, und die Länge der Rohre 1 bis 5 Meter.

Nach der Füllung leitet man bei vorgenannter Reaktionstemperatur Methanol unter den beschriebenen Reaktionsbedingungen durch den Reaktor. Das Methanol wird in der Regel in die Katalysatorschicht in einer Menge von 0,5 bis 10, vorzugsweise 0,8 bis 8 Kilogramm Methanol je Stunde und Liter Katalysator eingeleitet. Aus dem den Reaktor verlassenden Reaktionsgemisch trennt man in üblicher Weise, z. B. durch fraktionierte Destillation, den Formaldehyd oder seine 15- bis 50-gewichtsprozentige methanolische Lösung ab.

Der nach dem Verfahren der Erfindung herstellbare Formaldehyd ist Desinfektionsmittel, Gerbstoff, Reduktionsmittel und wertvoller Ausgangsstoff für die Herstellung von Kunstharzen, Klebmitteln, Kunststoffen und Hilfsmitteln in zahlreichen Industriesektoren. Bezüglich der Verwendung wird auf Ullmanns Encyklopädie der technischen Chemie, Band 7, Seite 670, verwiesen.

Die in den folgenden Beispielen angeführten Teile bedeuten Gewichtsteile. Sie verhalten sich zu den Volumenteilen wie Kilogramm zu Liter. Umsatz und Selektivität werden in der folgenden Form definiert.

$$\text{Umsatz } \% = \frac{\text{umgesetztes Methanol (Mol)}}{\text{zugeführtes Methanol (Mol)}} \times 100$$

$$\text{Selektivität } \% = \frac{\text{gebildeter Formaldehyd (Mol)}}{\text{umgesetztes Methanol (Mol)}} \times 100$$

## Beispiel 1

a) Katalysatorherstellung: 84 Teile Kupfer-(II)-oxid, 11,8 Teile Zinkoxid und 4,2 Teile Tellurdioxid werden mit 50 Teilen Wasser gut vermischt, bei 130°C getrocknet und zu Pillen von 3 mm Durchmesser und 30 mm Höhe verarbeitet.

b) Reaktion: 60 Volumenteile des so erhaltenen Katalysators werden in einen röhrenförmigen Quarzglasreaktor (∅ 3,2 cm) gegeben. 132 Teile/Stunde Methanol werden verdampft, in den Reaktor kontinuierlich eingeleitet und bei einer Temperatur von 550°C umgesetzt. Das Reaktionsgemisch wird kondensiert. Man erhält stündlich 20,2 Teile (Selektivität 76% der Theorie) Formaldehyd neben 0,5 Teilen Wasser, 79 Teilen Methanol. Der Umsatz beträgt 26 Prozent, der Anteil an mitgerissenem Tellur $< 0,5$ mg/kg Reaktionsgemisch.

Beispiel 2

a) Ein Katalysator wird aus 74,6 Teilen Kupfer-(II)-oxid, 15 Teilen Tellurdioxid und 10,4 Teilen Zinkoxid, wie in Beispiel 1 a beschrieben, hergestellt.

b) Die Umsetzung und Aufarbeitung erfolgt unter den in Beispiel 1 angegebenen Bedingungen. Man erhält stündlich 16,4 Teile (Selektivität 73% der Theorie) Formaldehyd neben 0,7 Teilen Wasser, 83 Teilen Methanol. Der Umsatz beträgt 22,6 Prozent, der Anteil an mitgerissenem Tellur $<0,5$ mg/kg Reaktionsgemisch.

Vergleichsbeispiel 3

a) Eine Mischung aus 50 Teilen Kupfer-(II)-oxid, 20 Teilen Ammoniumsulfat und 2 Teilen Zinkoxid wird gemahlen. Danach wird die pulverisierte Mischung durch Zugabe von 30 Teilen Wasser in eine Paste überführt und dann zu Tabletten mit einem Durchmesser von 3 mm und einer Dicke von 3 mm verformt. Anschließend werden die Tabletten in einer Wasserstoffatmosphäre 30 Minuten lang bei 200° C und dann 30 Minuten lang bei 600° C reduziert.

b) Unter Verwendung dieses Katalysators wird die Reaktion und Aufarbeitung unter den gleichen Bedingungen wie in Beispiel 1 durchgeführt. Man erhält stündlich 20,6 Teile (Selektivität 67% der Theorie) Formaldehyd neben 0,6 Teilen Wasser, 78 Teilen Methanol. Der Umsatz beträgt 29 Prozent, der Anteil an mitgerissenem Schwefel 126 mg/kg Reaktionsgemisch.

Vergleichsbeispiel 4

a) Aliquote Teile von 50,0 Teilen Kupfer-(III)-oxid, 7,0 Teilen Selendioxid und 7,0 Teilen Zinkoxid werden gemahlen und vermischt. 30 Teile Wasser werden zu dem Gemisch gegeben; man erhält eine Paste. Diese wird in Tabletten mit einem Durchmesser von 3 mm und einer Höhe von 3 mm verformt. Die so erhaltenen Tabletten werden in einem Wasserstoffgasstrom während einer Temperatur von 650° C während 30 Minuten reduziert.

b) Unter Verwendung dieses Katalysators wird die Reaktion und Aufarbeitung unter den gleichen Bedingungen wie in Beispiel 1 durchgeführt. Man erhält stündlich 19,4 Teile (Selektivität 68% der Theorie) Formaldehyd neben 1,0 Teilen Wasser, 79 Teilen Methanol. Der Umsatz beträgt 27 Prozent, der Anteil an mitgerissenem Selen 110 mg/kg Reaktionsgemisch.

Beispiel 5

a) Katalysatorherstellung

85,8 Teile Kupfer-(II)-oxid, 10 Teile Zinkoxid und 4,2 Teile Tellurdioxid werden mit 50 Teilen Wasser gut vermischt, bei 130° C getrocknet und zu Pillen von 3 mm Durchmesser und 3 mm Höhe verarbeitet.

b) Reaktion

60 Volumenteile des so erhaltenen Katalysators werden in einen röhrenförmigen Quarzglasreaktor (Ø 3,2 cm) gegeben. 132 Teile/Stunde Methanol werden verdampft, in den Reaktor kontinuierlich eingeleitet und bei einer Temperatur von 420° C umgesetzt. Das Reaktionsgemisch wird kondensiert. Man erhält stündlich 15,4 Teile (Selektivität 70% der Theorie) Formaldehyd neben 0,4 Teilen Wasser und 84 Teilen Methanol.

Die folgenden Beispiele zeigen die Lebensdauer der Katalysatoren.

Beispiel 6

Der Katalysator wird, wie in Beispiel 1 beschrieben, hergestellt und die Umsetzung unter den gleichen Bedingungen durchgeführt. Man entnimmt in Zeitabständen Proben und bestimmt den Gehalt der Reaktionskomponenten im Reaktionsgemisch. Die Ergebnisse sind in der nachfolgenden Tabelle 1 aufgeführt. Der Gehalt an Formaldehyd, Wasser unf Methanol ist in g/100 g Reaktionsgemisch, der Gehalt an Tellur in mg/kg Reaktionsgemisch angegeben.

Tabelle 1

| Reaktions-dauer (Stunden) | Gehalt an Formaldehyd | Wasser | Methanol | Gehalt an Tellur (mg/kg) | Umsatz (%) | Selektivität (%) |
|---|---|---|---|---|---|---|
| 8 | 14,8 | 0,8 | 84 | <0,5 | 19,2 | 79,8 |
| 18 | 14,0 | 0,9 | 85 | <0,5 | 20,5 | 77,4 |
| 36 | 13,1 | 0,6 | 86 | <0,5 | 18,2 | 73,0 |
| 72 | 8,4 | 0,6 | 90 | <0,5 | 13,1 | 66,3 |

Vergleichsbeispiel 7

Der Katalysator wird, wie im Vergleichsbeispiel 3 beschrieben, hergestellt und die Umsetzung wie in Beispiel 6 durchgeführt. Die Ergebnisse enthält Tabelle 2.

Vergleichsbeispiel 8

Der Katalysator wird, wie im Vergleichsbeispiel 4 beschrieben, hergestellt und die Umsetzung analog Beispiel 6 durchgeführt. Die Ergebnisse enthält Tabelle 3.

Die folgenden Beispiele zeigen die Regererierbarkeit der Katalysatoren.

Beispiel 9

Ein Katalysator wird, wie in Beispiel 5 beschrieben, hergestellt und die Umsetzung wie in Beispiel 6 durchgeführt. Nach jeweils 8 Stunden Reaktionsdauer wird der Katalysator bei 550 bis 600° C durch Überleiten von 200 000 Volumenteilen Luft während einer Stunde regeneriert. Die Ergebnisse sind in Tabelle 4 enthalten.

Vergleichsbeispiel 10

Ein Katalysator wird analog Vergleichsbeispiel 4 hergestellt. Man setzt analog Beispiel 9 um. Die Ergebnisse enthält Tabelle 5.

Tabelle 2

| Reaktions-dauer (Stunden) | Gehalt an Formaldehyd | Wasser | Methanol | Gehalt an Schwefel (mg/kg) | Umsatz (%) | Selektivität (%) |
|---|---|---|---|---|---|---|
| 9 | 21,5 | 0,6 | 77 | 170 | 28,3 | 72,4 |
| 18 | 16,3 | 0,5 | 82 | 30 | 24,1 | 65,5 |
| 36 | 12,3 | 1,0 | 86 | 7 | 20,8 | 57,1 |
| 72 | 8,4 | 2,5 | 88 | 3 | 19,7 | 40,2 |

Tabelle 3

| Reaktionsdauer (Stunden) | Gehalt an Formaldehyd | Wasser | Methanol | Gehalt an Selen (mg/kg) | Umsatz (%) | Selektivität (%) |
|---|---|---|---|---|---|---|
| 9 | 17,9 | 1,0 | 80 | 830 | 27,3 | 61,9 |
| 18 | 19,4 | 4,5 | 75 | 110 | 27,3 | 68,3 |
| 36 | 21,1 | 0,5 | 78 | 80 | 29,0 | 69,7 |
| 72 | 17,4 | 0,7 | 81 | 80 | 28,2 | 57,1 |

Tabelle 4

| Reaktionsdauer (Stunden) | Gehalt an Formaldehyd | Wasser | Methanol | Gehalt an Tellur (mg/kg) | Umsatz (%) | Selektivität (%) |
|---|---|---|---|---|---|---|
| 8 | 13,4 | 0,7 | 85 | <0,5 | 18,9 | 72,6 |
| 16 | 14,4 | 0,8 | 84 | <0,5 | 19,0 | 78,5 |
| 24 | 14,4 | 0,7 | 84 | <0,5 | 19,5 | 75,6 |
| 32 | 14,3 | 0,8 | 84 | <0,5 | 20,3 | 74,7 |
| 40 | 13,4 | 0,9 | 85 | <0,5 | 18,7 | 73,8 |
| 48 | 13,3 | 0,8 | 85 | <0,5 | 18,9 | 74,2 |
| 56 | 14,4 | 0,7 | 84 | <0,5 | 19,5 | 73,9 |
| 64 | 13,9 | 0,9 | 85 | <0,5 | 19,2 | 74,1 |
| 72 | 14,2 | 0,8 | 84 | <0,5 | 19,6 | 73,8 |
| 80 | 14,1 | 0,7 | 84 | <0,5 | 19,5 | 74,2 |

Tabelle 5

| Reaktionsdauer (Stunden) | Gehalt an Formaldehyd | Wasser | Methanol | Gehalt an Selen (mg/kg) | Umsatz (%) | Selektivität (%) |
|---|---|---|---|---|---|---|
| 8 | 23,0 | 1,5 | 75 | 130 | 30,6 | 68,0 |
| 16 | 24,0 | 1,3 | 74 | 42 | 34,8 | 65,8 |
| 24 | 21,2 | 1,1 | 77 | 27 | 30,4 | 66,8 |
| 32 | 19,0 | 1,1 | 79 | 6 | 26,0 | 72,6 |
| 40 | 20,6 | 0,9 | 78 | 8 | 28,9 | 69,9 |
| 48 | 18,6 | 1,0 | 80 | 4 | 27,0 | 67,0 |
| 56 | 21,0 | 1,0 | 77 | 7 | 30,5 | 65,6 |
| 64 | 15,8 | 0,9 | 82 | 3 | 24,1 | 64,2 |
| 72 | 15,5 | 0,9 | 82 | 3 | 24,7 | 61,0 |
| 80 | 14,9 | 0,8 | 83 | 3 | 23,4 | 62,0 |

**Patentanspruch**

Verfahren zur Herstellung von Formaldehyd durch Dehydrierung von Methanol in Gegenwart eines Katalysators bei erhöhter Temperatur, dadurch gekennzeichnet, daß die Umsetzung in Gegenwart von Kupfer, Zink und Tellur enthaltenden Katalysatoren bei einer Temperatur von 300 bis 750° C durchgeführt wird.

**Claim**

A process for the preparation of formaldehyde by dehydrogenating methanol in the presence of a catalyst at an elevated temperature, wherein the reaction is carried out in the presence of a catalyst containing copper, zinc and tellurium, at from 300 to 750° C.

**Revendication**

Procédé pour la preparation de formaldéhyde par déshydrogénation de méthanol en présence d'un catalyseur et à temperature élevée, caractérisé en ce que la réaction est exécutée à une température de 300 à 750° C en présence de catalyseurs contenant du cuivre, du zinc et du tellure.